# EUROPEAN PATENT APPLICATION

(11) **EP 3 560 551 A1**
(43) Date of publication of application: **30.10.2019**
(21) Application number: 18169395.3
(22) Date of filing: 26.04.2018
(51) Int. Cl.: A61N 1/10, A61B 5/00, A61K 9/70

(54) **A WEARABLE PATCH**

(71) Applicant: Koninklijke Philips N.V., 5656 AE Eindhoven (NL)
(72) Inventor: TALGORN, Elise Claude Valentine, 5656 AE Eindhoven (NL); SHRUBSOLE, Paul Anthony, 5656 AE Eindhoven (NL)
(74) Representative: de Haan, Poul Erik

(57) **Abstract**

A wearable patch (1) to be applied to a skin (20) of a body. The body has electrostatically pre-charged hairs. The wearable patch (1) comprises an adhesive layer (4) arranged at a contact side of the patch (1) with the skin (20) for applying the wearable patch (1) to the skin (20). The wearable patch (1) comprises an electrostatic element (5, 6) for aligning the electrostatically pre-charged hairs (10) along a specific direction (15) during applying the wearable patch to the skin, thereby making a contact area at the contact side of the patch with the skin more uniform.

## Description

### FIELD OF THE INVENTION

The invention relates to a wearable patch, a kit comprising the wearable patch and means for electrostatically pre-charging hairs of a body. The invention further relates to a method of using said wearable patch.

### BACKGROUND

Wearable skin patches are becoming more popular within the wearable market. They can be used for sports, drug-delivery, or patient monitoring, for example in electrocardiography (ECG or EKG), breathing rate monitoring, skin conductance measurements, etc. Wearable skin patches are ideal because they can be hidden under clothing, do not interfere with movement and therefore can be used to unobtrusively deliver drug to the body in case the patch is used as a therapeutic patch or alternatively to record more accurate data over a continuous and prolonged period of time in case the patch is used as a patient monitoring patch.

However, several general issues are associated with wearable skin patches.

There is a need of a good adhesion of the wearable skin patch with the skin. In patches delivering an active drug to the body, a good adhesion of the patch to the skin leads to a maximum absorption of the drug from the skin, and thus a patient can be better benefit from the drug. In patient monitoring patches, a good adhesion of the patch with the skin enhances a contact area of the patch with the skin, thereby improving accuracy of the monitoring. For this reason conductive gels or other type of conductive substances are applied to the skin prior to the application of the patch to enhance signal transmission from the body to the patient monitoring patch. In some cases, the part of the skin where the patch is applied, is shaved for improving further the contact of the patch with the skin. In fact hairs can prevent a good contact or reduce a surface contact of the patch with the skin.

Another issue associated with wearing the patch is discomfort. The patient often needs to wear the patch for a prolonged period of time. When the skin on which the patch is applied is shaved, hairs can regrow during time. Ingrown hairs, sometimes referred to as embedded hairs, may also grow below the patch and produce discomfort and in some cases also to infections and scarring of the skin.

The patch may cause discomfort even upon removal. It is well known the sensation that one proves when an adhesive patch is removed from a hairy skin: the hairs are pulled by the adhesive of the patch causing distress to the person wearing the patch. There is thus a need for an improved patch.

### SUMMARY OF THE INVENTION

It would be advantageous to have a patch that enhances adhesion of the wearable patch with the skin in presence of hairs.

One aspect of the invention provides a wearable patch to be applied to a skin of a body. The body has electrostatically pre-charged hairs. The wearable patch comprises an adhesive layer arranged at a contact side of the patch with the skin for applying the wearable patch to the skin. The wearable patch comprises an electrostatic element for aligning the electrostatically pre-charged hairs along a specific direction during applying the wearable patch to the skin.

Patches for medical, cosmetical or monitoring purposes are often placed on body parts like arms, torso, legs, which are covered, especially in men, with hairs. The hairs entangle under the patch and results in an intermediate layer between the patch and the skin that diminishes patch adhesion with the skin, thereby hampering effectiveness of the patch.

Advantageously, with the patch of the invention, hairs are all aligned along the specific direction during application of the patch. If the electrostatic element and the hairs are charged with the same polarity, the hairs are repelled by the electrostatic element during applying the patch. If the electrostatic element and the hairs are charged with opposite polarity, the hairs are attracted by the patch. In both cases, the electrostatic element is arranged to align the hairs along the specific direction. Under the effect of the attraction or repulsion of the electrostatic element, the hairs extend along their length along the specific direction such that hairs are less entangled. There are less chances that a layer of entangled hairs is formed between the skin and the patch preventing contact between the patch and the skin. A contact area at the contact side of the patch with the skin is more uniform. Adhesion of the wearable patch with the skin is thus improved.

Since contact between the patch and the skin is enhanced even in the presence of hairs, shaving the skin in the application area of the patch is not required. Shaving the skin can cause irritation or scratches of the skin. Furthermore, if the patch is worn for a prolonged period of time and the skin is shaved, ingrown hairs can grow under the patch causing discomfort and in some cases also infections. The patch of the invention can be easily applied to the skin without that the skin is shaved, thereby preventing the above mentioned common issues associated with shaving the skin.

In an embodiment, the electrostatic element is arranged at an end portion of the wearable patch.

If the electrostatic element is arranged at the end portion of the patch, application of the patch in presence of hairs is more effective because the hairs are progressively more attracted or repelled along the direction of application towards the end portion of the patch during application of the patch. If the electrostatic element is distributed all over the patch, hairs may still entangle during application because they would be attracted or repelled towards the patch at any portion of the patch closer to the skin. Providing the electrostatic element at an end portion of the patch allows for a better alignment of the hairs along the direction of application especially for the hairs distributed under the patch further away from the end portion. There are thus fewer chances that hairs are entangled under the patch further away from the end portion.

In another embodiment, the electrostatic element comprises stripes of electrostatic material aligned with the specific direction. Extension of the hairs along their length can be facilitated by the aligned stripes of electrostatic material, thereby improving effectiveness of hairs alignment. Alignment of hairs in any other direction different from the direction of application is prevented, thereby enhancing the surface contact between the patch and the skin.

In an embodiment, the wearable patch is arranged to be applied to the skin and/or to be removed from the skin along the specific direction.

For example, the wearable patch has a shape for indicating the specific direction. For example, the patch may have an asymmetrical shape, like an arrow shape to indicate the specific direction.

For example, in some other embodiments, the wearable patch may comprise one or more of a mark or text stamped on the patch for indicating application and/or removal of the patch, and an ergonomically shaped feature for facilitating application and/or removal of the patch. For example, the wearable patch may comprise stamped arrows to indicate a direction of application or stamped words like "apply" and/or "remove" to indicate a side of the patch which is being applied to the skin or removed from the skin.

Another aspect of the invention provides a kit. The kit comprises a patch and means for electrostatically charging the hairs. For example, the kit is provided with a piece of a triboelectric material which can be rubbed onto the skin to electrostatically pre-charge the hairs. The patch may be applied subsequently.

Another aspect of the invention provides a method of using the patch of the invention. The method comprises electrostatically pre-charging the hairs of the body, applying the patch along the specific direction along which the electrostatically pre-charged hairs are aligned, and removing the patch along the same specific direction. For example, the specific direction may be a direction of application of the patch.

Removing the patch along the direction of application lessen pain during removal of the patch because hairs are not pulled away by the adhesive layer. Removal of the patch occurs from a root side of the hairs and not from a tip side which makes the step of removal less uncomfortable.

### BRIEF DESCRIPTION OF THE DRAWINGS

Further details, aspects, and embodiments of the invention will be described, byway of example only, with reference to the drawings. Elements in the figures are illustrated for simplicity and clarity and have not necessarily been drawn to scale. In the Figures, elements which correspond to elements already described may have the same reference numerals. In the drawings,
Figure 1A schematically shows a cross sectional view of an example of a body's skin with hairs,
Figure 1B schematically shows a cross sectional view of an example of a wearable patch to be applied to a skin of a body,
Figure 1C schematically shows a cross sectional view of an example of a wearable patch applied to a skin of body,
Figure 1D schematically shows a cross sectional view of an example of a wearable patch to be removed from a skin of a body,
Figure 2 schematically shows a top view of an example of a wearable patch,
Figure 3 shows a cross sectional view of a wearable patch taken along the line III-III of Figure 2,
Figure 4 shows a bottom view of an example of a wearable patch,
Figure 5 schematically shows a bottom view of an example of a wearable patch,
Figure 6 schematically shows a flow diagram for a method of using a wearable patch.

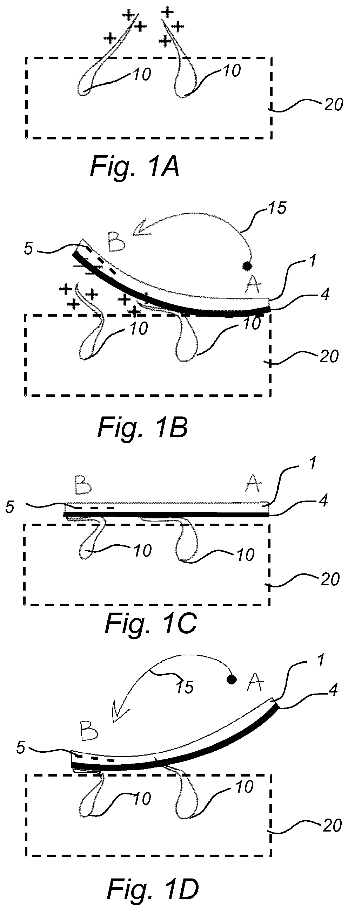

### List of Reference Numerals in figures 1A-5:

- 1, 2, 3, 13: a wearable patch
- 4: an adhesive layer
- 5, 6: an electrostatic element
- 7, 8: an ergonomically shaped feature
- 9: a triboelectric material
- 10: a hair
- 11, 12: a stamped text
- 15, 16, 18: a specific direction
- 17: stripes of electrostatic material
- 20: a skin of a body

### DETAILED DESCRIPTION OF PREFERRED EMBODIMENTS

While this invention is susceptible of embodiment in many different forms, there are shown in the drawings and will herein be described in detail one or more specific embodiments, with the understanding that the present disclosure is to be considered as exemplary of the principles of the invention and not intended to limit the invention to the specific embodiments shown and described.

Further, the invention is not limited to the embodiments, and the invention lies in each and every novel feature or combination of features described herein or recited in mutually different dependent claims.

Figure 1A schematically shows a cross sectional view of an example of a body's skin 20 with hairs 10. Hairs 10 are electrostatically pre-charged, for example with a negative charge which is indicated in Figure 1 with the sign minus. However, hairs 10 may be electrostatically pre-charged also with a positive charge. Hairs 10 may be electrostatically pre-charged in any manner suitable for the specific implementation.

In an embodiment, hairs 10 may be electrostatically pre-charged by friction with, for example, a triboelectric material. The triboelectric material may be, for example, a rubber piece or a plastic piece. In some embodiments a rubbing glass with fur, or a plastic comb passing through hairs 10 may electrostatically pre-charge hairs 10.

Figure 1B schematically shows a cross sectional view of an example of a wearable patch 1 to be applied to a skin 20 of a body with electrostatically pre-charged hairs 10.

Wearable patch 1 comprises an adhesive layer 4 for applying wearable patch 1 to skin 20. Wearable patch 1 further comprises an electrostatic element 5 for aligning the electrostatically charged hairs along a specific direction 15 during applying the patch.

In an embodiment, wearable patch 1 may be arranged to be applied along the specific direction, which in the following will be referred to as a direction of application of the patch with the skin.

In the example shown in Figure 1B, direction of application 15 is generically indicated by the letters A and B. A person starts with applying patch 1 from a side of patch 1 indicated with letter A and finishes applying patch 1 at a side of patch 1 indicated with letter B.

If hairs 10 are positively charged, then the electrostatic element may comprise a material specifically chosen such that it attracts negatively charged objects. Alternatively, if both the hairs and the electrostatic element are charged with the same polarity, the patch may repel the hairs. In both cases the effect achieved is that by

In an embodiment, electrostatic element 5 comprises glass or one or more polymers selected from the group of polymers comprising cellulose plastics, polyvinyl chloride polypropylenes, polystyrenes, polyurethanes, polycarbonates, polyacrylics, polyolefines, polyesters, polyethylenes and ethylene vinyl acrylates.

Before application, a person may place patch 1 in close proximity with skin 20 such that electrostatically charged hairs 10 are initially aligned all in the same direction. The person may make a movement with patch 1 at some distance from skin 20 in the direction of application such that hairs 10 become aligned along the direction of application. For example, the person applying the patch may begin to move patch 1 at some distance from the skin from a skin area outside the application area and approach slowly the application area in the indicated direction of application. Hairs 10 are thus progressively attracted or repelled along the direction of application of patch 1. Contact with the skin can then be made at side of patch 1 indicated with letter A and progressively following the direction of application also at side of patch 1 indicated with letter B as schematically shown in Figure 1C.

Patches are often placed on body parts like arms, torso, legs, which are covered, especially in men, with hairs. The hairs entangle below the patch and results in an intermediate layer between the patch and the skin that diminishes patch adhesion with the skin and hampers effectiveness of the patch.

Advantageously, with patch 1 of the invention, under attraction or repulsion of the electrostatic element, hairs can extend along their length in the specified direction and be thereby aligned. Alignment of hairs 10 along the application direction makes a contact area of patch 1 with skin 20 more uniform. As a consequence hairs 10 are less entangled. There are less chances that a layer of entangled hairs is formed between the skin and the patch preventing contact between the patch and the skin. Adhesion of the wearable patch with the skin is thus improved.

Since contact between patch 1 and skin 20 is enhanced even in the presence of hairs, shaving the skin in the application area of the patch is not required. Patch 1 can be easily applied to the skin without that the skin is shaved. Shaving the skin can cause irritation or scratches of the skin.

In an embodiment, the electrostatic element is arranged at an end portion of the wearable patch in the specific direction. For example, as shown in Figures 1B-1D, a person may start applying patch 1 from an opposite side of said end portion and finish applying patch 1 at the end portion..

If the electrostatic element is arranged at the end portion of the patch and application of the patch finishes at this end portion, application of the patch in presence of hairs is more effective because the hairs are progressively more attracted or repelled along the direction of application towards the end portion during application of the patch. If the electrostatic element is distributed all over the patch, hairs may still entangle during application because they would be attracted or repelled towards the patch at any portion of the patch closer to the skin. Providing the electrostatic element only at an end portion of the patch allows for a better alignment of the hairs along the direction of application especially for the hairs distributed under the patch further away from the end portion comprising the electrostatic element. There are thus fewer chances that hairs are entangled under the patch further away from the end portion.

In an embodiment shown later, the electrostatic element comprises stripes of electrostatic materials aligned with the direction of application of the patch.

Alignment of the stripes of electrostatic material with the direction of application improves effectiveness of hairs alignment. Alignment of hairs in any other direction different from the direction of application is prevented, thereby enhancing the surface contact between the patch and the skin.

Wearable patch 1 may be any kind of patches.

Patch 1 may be for example a patch for applying an active substance to skin 20. Such patches are sometimes referred to as transdermal patches. The substance enters a bloodstream of the body transdermally, i.e. through skin 20. The substance, once the patch is applied to the skin, is absorbed by the skin and distributed through the body via the bloodstream. The substance may be a therapeutic substance or a cosmetic substance.

Advantages of using transdermal patches are that they are safe, reliable and pain-free. They are especially suitable for patients who have difficulty swallowing and for releasing active substances with low oral bioavailability. Doses can be controlled by controlling a release rate of the substance. Contact of transdermal patch with the skin for a prolonged period of time is critical for ensuring that correct doses of the substance are releases to the skin. The patch of the invention enhances the contact of the patch with the skin in presence of hairs which is beneficial for maintaining the desired release rate of the substance over the time period during which the patch is applied to the skin.

In an embodiment, the adhesive layer used for applying the patch to the skin comprises the substance to be released to the skin. A single adhesive layer can thus be used for attaching the patch to the skin and for storing the active substance.

In another embodiment, the patch may further comprise a substance reservoir layer for storing the substance to be applied to the skin. The substance may be an active substance as explained above, for example a drug solution or suspension. The layer may be a matrix, for example a semisolid matrix where the substance is stored and from where the substance is released to the skin.

In an embodiment, the patch may comprise a rate controlling membrane layer for controlling release of the substance. The rate controlling membrane is for example made of a polymer like vinyl acetate on one surface of the membrane. The substance release rate depends on the membrane permeability and diffusion of drug molecules.

However, other different types of patches than patches only suitable for releasing an active drug are also contemplated.

For example, in an embodiment, patch 1 may comprise a sensor for monitoring a vital sign of the body.

Patch 1 may thus be used to monitor a vital signal, like heart rate, breathing rate, skin conductance. For example, the patch of the invention may be used in electrocardiography (ECG or EKG) for recording an electrical activity of the heart over a (prolonged) period of time.

To this purpose the patch of the invention may comprise electrodes (not shown in the Figures) which upon application of patch with the skin are in contact with the skin. These electrodes detect electrical changes on the skin that arise from the heart muscle's electrophysiological pattern of depolarizing and repolarizing during each heartbeat. It is important that contact of the electrodes with the skin is maintained over the whole measurement period. The patch of the invention may enhance the contact of the electrodes with the skin in presence of hairs, thereby improving accuracy of detection of the electrical changes.

In an embodiment, the patch may further comprise a release liner layer covering the adhesive layer. The release liner layer is removed shortly before application of the patch into the skin. The release liner layer prevents that the contact side of the patch is sticky before application of the patch. With the release liner, the adhesive layer of the patch can maintain its sticking properties until application.

Figure ID schematically shows a cross sectional view of an example of a wearable patch to be removed from a skin of a body. Patch 1 may be removed along a direction of removal equal to the direction of application. In the example shown in Figure 1D, the direction of application is from A to B and the direction of removal is also from A to B in the same direction of application. Thus the patch may be arranged to be removed along the same direction of application.

When the patch is applied and removed along the same direction, removal of the patch is less uncomfortable because hairs are pulled less further away from the skin than when the patch is removed along a direction opposite to the direction of application, i.e. in the example shown from B to A.

Advantageously, a person may benefit from the inventive patch not only when the patch is applied in presence of hairs, thus increasing the contact area of the skin with the patch, but also when the patch is removed from the skin, making the removal of the patch less uncomfortable.

In an embodiment, the patch may comprise at least a guiding element for indicating the direction of application.

In an embodiment, the guiding element comprises one or more of a mark or text stamped on the patch for indicating application and/or removal of the patch, and an ergonomically shaped feature for facilitating application and/or removal of the patch.

In an embodiment, letters or numbers may be stamped on the patch to indicate a person applying the patch to the skin that he should start with applying the patch with a side of the patch indicated with a specific letter or number and finish with applying the patch with a side of the patch indicated with a different letter or number. Alternatively, in an embodiment, the patch may have a particular shape, for example an arrow shape or an asymmetrical shape, indicating the direction of application of the patch.

Alternatively, in another embodiment, the patch may comprise one or more ergonomically shaped features embedded in the patch. For example, a first feature may be a concave shaped portion of the patch against which a person can press the patch to the skin with a finger's tip. A second feature may be a patch extension portion sticking out of the adhesive layer for enabling a person to grab the extension and remove the patch from the skin.

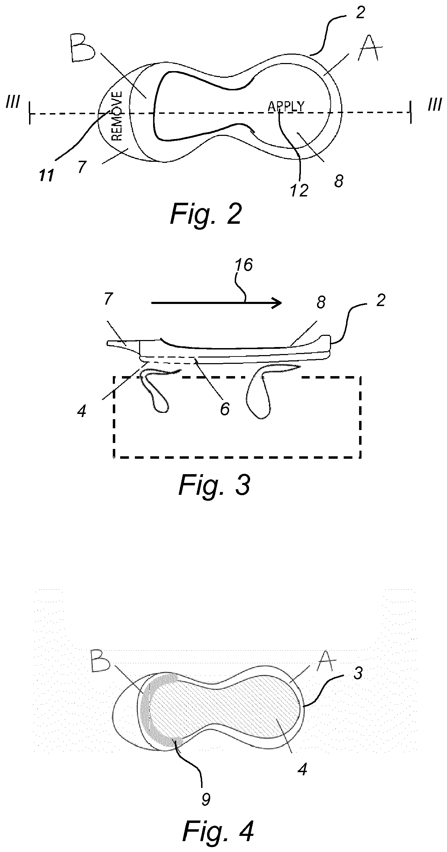

Figure 2 schematically shows a top view of an example of a wearable patch 2.

Figure 3 shows a cross sectional view of wearable patch 2 taken along the line III-III of Figure 2.

Wearable patch 2 comprises a stamped text 11, "apply", and a stamped text 12, "remove", to indicate a side of application and removal of wearable patch 2, respectively. Furthermore, as shown in the cross sectional view of Figure 3, patch 2 comprises ergonomically shaped features 7 and 8 to facilitate removal and application of patch 2, respectively.

Feature 8 is a regression on upper side of patch 2 which substantially extends along the length of patch 2. Regression 8 has a width of a fingertip so as to allow a fingertip to press on regression 8 to facilitate application of patch 2 along its length.

Feature 7 is a protrusion of patch 2. Protrusion 7 is shaped so as to allow a person to grab the protrusion with e.g. two fingers, allowing the person to pull patch 2 from the skin, e.g. along a predetermined direction of removal.

Text 11, 12 and ergonomically shaped features 7 and 8 are example of guiding elements indicating the direction of application and/or removal of patch 2. Different text may be stamped on the patch to indicate the direction of application and removal. The patch may have a specific shape for indicating the direction of application and/or removal: for example an arrow shape, a double asymmetrical arrow shape, etc. Similarly, different ergonomically shaped features may be embedded in the patch as long as they can facilitate application and removal of the patch.

Wearable patch 2 further comprises electrostatic element 6. Electrostatic element 6 may be arranged to repel the electrostatically pre-charged hairs. For example the hairs may be pre-charged with the same polarity of the electrostatic element. A person may move patch 2 such that hairs are aligned along a direction opposite to the alignment direction shown in Figures 1B-1D, i.e. in this example from left to right or from B to A. Differently from the exemplary embodiment of Figures 1B-1D, patch 2 maybe applied at once by making contact on both sides A and B at the same time. Application at both sides A and B may be facilitated by regression 8 which extends substantially towards side A and side B of patch 2. During application, alignment of the hairs from B to A in Figure 2, i.e. along specific direction 16 in Figure 3, is thus maintained. During removal, protrusion 7 facilitates removal of the patch in the same direction 16 of alignment, i.e. from B to A in Figure 2, so as to minimize discomfort which would be caused by pulling the hairs from the tip side.

Figure 4 shows a bottom view of an example of a wearable patch 3. Wearable patch 3 comprises adhesive layer 4, e.g. filling most of the bottom surface of patch 3. Adhesive layer 4 may be covered with a release liner (not shown in Figure 4). The release liner may be removed shortly before application of patch 3.

Wearable patch 3 comprises a triboelectric material 9 for electrostatically per-charging the hairs. In Figure 4 triboelectric material 9 partially surrounds adhesive layer 4 at one side of wearable patch 3 indicated with the letter B. However, the triboelectric material may be placed in any other side of the patch, e.g. could completely surround adhesive layer. Alternatively, the release liner itself may be made of a triboelectric material.

Triboelectric material 9 may thus be used to electrostatically charge the hairs before applying patch 3 onto the skin and before, for example, removing the release liner from adhesive layer 4. Triboelectric material 9 may be rubbed onto the hairs, after which the release liner is removed from the patch for applying the patch onto the skin.

In an embodiment, the electrostatic element used for attracting the hairs along the direction of application is made of a triboelectric material. For example, the triboelectric material may be used to electrostatically pre-charge the hairs before application of the patch and then to electrostatically attract or repel the electrostatically pre-charged the hairs along the direction of application of the patch. In the example shown with Figure 4, triboelectric material 9 may be used for both mentioned purposes. In this way, a single electrostatic element embedded in the patch can be used for two difference functions, namely for the function of electrostatically pre-charging the hairs and for the function of attracting or repelling the electrostatically pre-charged hairs along the direction of application.

In an alternative embodiment, the electrostatic element comprises the release liner. Removal of the release liner may generate the electrostatic charges on the patch attracting or repelling the electrostatically pre-charged hairs. In this embodiment, the electrostatic element may be embedded with the release liner preventing the addition of a separate element arranged to electrostatically attract or repel the hairs.

In yet an alternative embodiment, the patch may be complemented with a kit comprising the patch and means to electrostatically pre-charge the hairs. For example, the kit is provided with a piece of a triboelectric material which can be rubbed onto the skin to electrostatically charge the hairs. The material can be any material suitable for electrostatically charging the hairs as mentioned in some of the embodiments described above. The piece of material can be a soft piece like for example a tissue piece, a fur or a rigid piece like for example a plastic comb. A separate means for electrostatically charging the hairs may facilitate pre-charging of the hairs without the use of the patch, thereby preventing that the patch is damaged by rubbing the patch with the skin. Use of a separate piece of triboelectric material arranged to electrostatically pre-charge the hairs may also improve the electrostatically pre-charging of the hairs. The piece of triboelectric material may be provided with a large rubbing surface thereby allowing a more effective electrostatic pre-charging of the hairs.

In an embodiment, the electrostatic element comprises a charge storage device. For example, the charge storage device may be arranged to store a charge of opposite or equal sign to a charge to which the hairs are electrostatically pre-charged.

For example, the electrostatic element may comprise a battery or a capacitive device capable to store charges with a certain polarity. The battery or capacitive device may be embedded with electrodes of the patch. The patch of this embodiment may be of the type including a sensor for monitoring a vital signal as described with reference to some of the previous embodiments. In case a capacitive device is used, this may be pre-charged by a local battery embedded in the patch or connected to the patch via e.g. the electrodes and electrical wires connected at one side to the electrodes and at the other side to the battery. Alternatively, the capacitive device may be supplied wirelessly, for example via inductive charging.

The charge storage device may be negatively charged and the hairs positively charged. Interaction between the negatively charged charge storage device and the positively charged hairs create a reduction in the negative potential of the negatively charged charge storage device thereby causing attraction of the electrostatically pre-charged hairs. In an embodiment, the wearable patch comprises a charge detector for detecting a charge of the electrostatically pre-charged hairs, and a processor for processing the monitored vital signal based on the detected charge.

For example, the charge detector may comprise a capacitive element which can detect the charge of the electrostatically charged hairs. For example, the capacitive element is pre-charged. Once the capacitive element is placed in close proximity to the electrostatically pre-charged hairs, the capacitive element changes its charge amount. The change in the charge amount may be an indication of the charge of the electrostatically pre-charged hairs. The patch or system including the patch used for monitoring the vital sign may comprise a processor coupled to the capacitive element for retrieving such change of charge. The charge change is translated by the processor into an estimated amount of hairs covered by the patch. For example, an average charge of a single hair may be known, for example via previously performed tests or experiments aiming at monitoring the electrostatically average charges of hairs. By using the known average charge of a single air, the processor may translate the detected charge change into the amount of hairs covered by the patch.

The processor may additionally comprise an algorithm to correct the measurement of the monitored vital signal based on the amount of hairs covered by the patch. For example, it is known that the hairs reduce the surface contact area between the patch and the skin, making the measurement of the vital signal less accurate, thereby reducing a signal to noise ratio of the measurement. The algorithm may compensate for the less accurate reading by using the information on the estimated amount of hairs present between the patch and the skin. For example, a predetermined amount of hairs may be associated to a predetermined signal to noise reduction. An error correction algorithm can take into account of this signal to noise reduction caused by the presence of hairs and compensate for it.

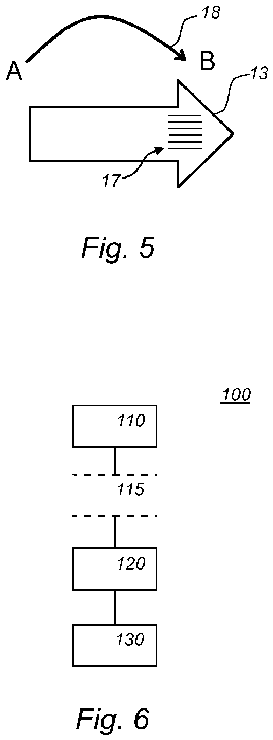

Figure 5 schematically shows a plane view of an example of a wearable patch 13. Wearable patch 13 comprises an adhesive layer (not shown in Figure 5) for applying wearable patch 13 to the skin. Wearable patch 13 has an arrow shape to indicate for example a specific direction 18 of alignment of the hairs during application of the patch. In this example, specific direction 18 of alignment corresponds to a direction of application of the patch with the skin. In other words, patch 13 may be first applied at side A and attached along its length so as that it may be finished to be applied at side B. Wearable patch 13 comprises stripes 17 of electrostatic material aligned along the direction of application. Stripes 17 may be embedded in patch 13 or arranged on a bottom surface. In this example, stripes 17 are arranged at the arrow end. However, the stripes may be covering the full length of the patch. The stripes 17 facilitate alignment of the hairs along the direction indicated by the arrow when wearable patch 13 is being applied to the skin or when wearable patch 13 is placed in close proximity with the hairy skin.

Figure 6 schematically shows a flow diagram for a method 100 of using a wearable patch. The wearable patch can be any of the patches 1, 2 and 3 described with reference to the Figures 1-5. The method can be also applied to a kit comprising the patch and a separate element for electrostatically charging the hairs.

Method 100 comprises electrostatically pre-charging 110 the hairs of a body, applying 120 the patch along a specific direction, for example a direction of application of the patch and removing 130 the patch along the same specific direction. Hairs are attracted or repelled along a specific direction, for example from A to B as shown in the embodiment of Figure 1B. Removing the patch along the same direction of application, for example also from A to B as shown in Figure ID, prevents to pull the hairs, making the removal less uncomfortable and annoying for the person using the patch.

Electrostatically pre-charging 110 hairs of the body may optionally comprise rubbing the patch onto the skin. For example, the patch may comprise a triboelectric material suitable to electrostatically charge the hairs by rubbing it with the skin.

Method 100 may optionally comprise after electrostatically pre-charging 110 the hairs, approaching 115 the patch to the skin such that the electrostatically charged hairs are initially attracted or repelled all in the same direction. For example, the person applying the patch may make a movement with patch 1 at some distance from skin 20 in a specific direction, for example a direction of application, for example parallel to the skin, such that the hairs become aligned along said direction. For example, the person applying the patch may begin to move the patch at some distance from the skin from a skin area outside the application area and approach slowly the application area in the indicated direction of application.

Contact with the skin can then be progressively made following the specific direction as described with reference to the Figure 1B and 1C.

It should be noted that the above-mentioned embodiments illustrate rather than limit the invention, and that those skilled in the art will be able to design many alternative embodiments. For example, the patches 1, 2, 3 shown with reference to the Figures 1A-5 have all a specific elongated shape. However, any other shape is possible, circular, elliptical, polygonal, elongated or not elongated as long as the direction of application is indicated. In the claims, any reference signs placed between parentheses shall not be construed as limiting the claim. Use of the verb "comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

In the claims references in parentheses refer to reference signs in drawings of embodiments or to formulas of embodiments, thus increasing the intelligibility of the claim. These references shall not be construed as limiting the claim.

## Claims

1. A wearable patch (1) to be applied to a skin (20) of a body, the body having electrostatically pre-charged hairs (10), the wearable patch comprising:
- an adhesive layer (4) arranged at a contact side of the patch (1) with the skin (20) for applying the wearable patch (1) to the skin (20), wherein the wearable patch (1) comprises an electrostatic element (5) for aligning the electrostatically pre-charged hairs (10) along a specific direction (15) during applying the wearable patch (1) to the skin (20).

2. A wearable patch (1, 2) according to claim 1, wherein the electrostatic element (5, 6) is arranged at an end portion of the wearable patch (1, 2) in the specific direction (15, 16).

3. A wearable patch (13) according to any of the preceding claims, wherein the electrostatic element comprises stripes (17) of electrostatic material aligned with the specific direction (18).

4. A wearable patch (13) according to any of the previous claims, having a shape for indicating the specific direction (18).

5. A wearable patch (1, 2, 3, 13) according to any of the preceding claims, arranged to be applied to the skin and/or to be removed from the skin along the specific direction.

6. A wearable patch (2) according to claim 5, wherein the wearable patch (2) comprises one or more of
- a stamped mark or text (11, 12) on the patch for indicating application and/or removal of the patch, and
- an ergonomically shaped feature (7, 8) for facilitating application and/or removal of the patch.

7. A wearable patch according to any of the previous claims, wherein the electrostatic element comprises glass or one or more polymers selected from the group of polymers comprising cellulose plastics, polyvinyl chloride polypropylenes, polystyrenes, polyurethanes, polycarbonates, polyacrylics, polyolefines, polyesters, polyethylenes and ethylene vinyl acrylates.

8. A wearable patch according to any of the previous claims, wherein the electrostatic element comprises a charge storage device.

9. A wearable patch according to any of the previous claims, comprising a sensor for monitoring a vital signal of the body.

10. A wearable patch according to claim 9, comprising a charge detector for detecting a charge of the electrostatically pre-charged hairs, and a processor for processing the vital signal based on the detected charge.

11. A wearable patch according to any of the previous claims, comprising a substance reservoir layer for storing a substance to be applied to the skin.

12. A wearable patch (3) according to any of the claims 1 to 11, comprising a triboelectric material (9) for electrostatically pre-charging the hairs.

13. A wearable patch (3) according to claim 12, wherein the electrostatic element is made of the same triboelectric material.

14. A kit comprising a patch according to any of the claims 1 to 11 and means for electrostatically pre-charging the hairs.

15. Method of using a patch according to any one of the claims 1 to 13, the method comprising:
- electrostatically pre-charging (110) hairs of a body,
- applying (120) the patch along the specific direction,
- removing (130) the patch along the specific direction.
